# EUROPÄISCHE PATENTANMELDUNG

(11) **EP 1 206 945 A1**
(43) Veröffentlichungstag der Anmeldung: **22.05.2002**
(21) Anmeldenummer: 01126922.2
(22) Anmeldetag: 13.11.2001
(51) Int. Cl.: A61L 27/50, A61L 29/18, A61L 31/18

(54) **Medizinische Arbeitsmittel mit paramagnetischen / radio-opaquen Zusätzen**

(30) Priorität: 17.11.2000 DE 10057038
(71) Anmelder: REHAU AG + Co, 95111 Rehau (DE)
(72) Erfinder: Dolla, Andreas, 95111 Rehau (DE); Ziembinski, Ralf,Dr, 95111 Rehau (DE)

(57) **Zusammenfassung**

Die Erfindung betrifft medizinische Arbeitsmittel, insbesondere Katheter, Schläuche, Rundschnüre, Formteile aus polymeren Materialien, wobei diese Arbeitsmittel eine besondere Ausstattung zu ihrer Erkennung in der Kernspintomographie aufweisen.

Dies wird dadurch erreicht, dass in das polymere Material vor der eigentlichen Formgebung eine definierte Menge wenigstens einer paramagnetischen Metallverbindung und/oder eines paramagnetischen Metalles eingemischt ist.

## Beschreibung

Die Erfindung betrifft medizinische Arbeitsmittel, insbesondere Katheter, Schläuche, Rundschnüre, Formteile aus polymeren Materialien, wobei diese Arbeitsmittel eine besondere Ausstattung zu ihrer Erkennung in der Kernspintomographie aufweisen.

Derartige medizinische Arbeitsmittel werden zur Durchführung von zahlreichen therapeutischen und diagnostischen Eingriffen in der Medizin verwendet. Zur genauen Überwachung derartiger Eingriffe müssen die verwendeten medizinischen Arbeitsmittel jedoch vom behandelnden Mediziner jederzeit in ihrer Medizin und Lage kontrollierbar sein. Dies ist insbesondere für den Erfolg derartiger Eingriffe als auch für die Sicherheit der zu behandelnden Patienten von elementarer Bedeutung.

Eines der hierfür häufig verwendeten Verfahren ist neben dem Röntgen die Kernspintomographie.
Nach dem bekannten Stand der Technik werden die medizinischen Arbeitsmittel für die Kontrolle im Kernspintomographieverfahren mit speziellen metallischen Elementen wie beispielsweise Metalldrähten versehen, die wiederum in der Kernspintomographie sichtbar sind.
Eine weitere Möglichkeit nach dem bekannten Stand der Technik besteht in dem Einsatz von medizinischen Arbeitsmitteln, welche aus metallischen Werkstoffen hergestellt werden, welche in der Kernspintomographie sichtbar sind.

Nachteilig bei diesen medizinischen Arbeitsmitteln wird jedoch gesehen, dass aufgrund der eingesetzten Werkstoffe die Produkte sehr steif und starr sind und somit nur einen eingeschränkten Anwendungsbereich auf bestimmte therapeutische oder diagnostische Eingriffe haben.
Es hat sich jedoch auch herausgestellt, dass bei der Verwendung derartiger medizinischer Arbeitsmittel eine gewisse Sichtbarkeit in der Kernspintomographie erreichbar ist, welche jedoch nicht ausreichend bzw. zu stark ausgeprägt ist, um die Position exakt zu bestimmen und die außerdem den zusätzlichen Nachteil aufweist, dass sie nicht optimal ist.

Derartige medizinische Arbeitsmittel weisen jedoch auch noch den Nachteil auf, dass sie einer recht aufwendigen und kostenintensiven Herstellung bedürfen, welche sich negativ im Preis-Leistungs-Verhältnis dieser Produkte niederschlägt.

Bei der Verwendung medizinischer Arbeitsmittel, in die beispielsweise ein Draht eingebracht ist, welcher in der Kernspintomographie sichtbar ist, besteht der Nachteil, dass nur der Draht als solches sichtbar ist, jedoch die Geometrie des medizinischen Arbeitsmittels, beispielsweise eines Katheters oder eines Einführungsbesteckes, welche eine größere Ausdehnung aufweisen, nicht eindeutig darstellbar ist.

Weiterhin sind aus dem Stand der Technik medizinische Arbeitsmittel bekannt, in denen beispielsweise die verwendeten Katheter aus sogenannten Mehrlumenschläuchen hergestellt sind, wobei in ein Lumen ein in der Kernspintomographie sichtbarer Werkstoff beinhaltet ist, welcher beispielsweise eine Flüssigkeit sein kann, die den großen Nachteil aufweist, dass bei einem Flüssigkeitsaustritt infolge von Undichtigkeit der medizinische Eingriff zum Scheitern verurteilt ist, die Patientensicherheit gefährdet ist und die zudem noch einen sehr hohen Herstellungsaufwand, verbunden mit hohen Kosten aufweisen.

Hier setzt die Erfindung ein, die es sich zur Aufgabe gestellt hat, die Nachteile des bekannten Standes der Technik zu vermeiden und medizinische Arbeitsmittel aufzuzeigen, die kostengünstig und wirtschaftlich herstellbar sind, die in der Kernspintomographie eine optimale Abbildung liefern und die sowohl in der Kernspintomographie als auch im Röntgen vollflächig sichtbar sind.

Erfindungsgemäß wird dies dadurch gelöst, dass in das polymere Material vor der eigentlichen Formgebung eine definierte Menge wenigstens einer paramagnetischen Metaliverbindung und/oder eines paramagnetischen Metalles eingemischt ist.

Vorteilhaft wird hier gesehen, dass durch die erfindungsgemäßen medizinischen Arbeitsmittel jederzeit eine problemlose und optimale Sichtbarkeit in der Kernspintomographie erreicht werden kann, wobei die Herstellung dieser medizinischen Arbeitsmittel nach den bekannten Verfahren der Polymerherstellung, bzw. Thermoplastaufbereitung bzw. -verarbeitung erfolgen kann.

Es wird weiter vorteilhaft gesehen, dass die Menge der paramagnetischen Metallverbindung und/oder eines paramagnetischen Metalles zwischen 0,1 und 80 Gewichtsprozent liegt, vorzugsweise zwischen 1 und 40 Gewichtsprozent.
Es wurde überraschend festgestellt, dass durch die Variation des Anteils der paramagnetischen Metallverbindung und/oder eines paramagnetischen Metalles die Sichtbarkeit in der Kernspintomographie in Abhängigkeit sowohl des verwendeten Werkstoffes als auch der Geometrie der erfindungsgemäßen Arbeitsmittel jederzeit einstellbar und optimierbar ist.

Es wurde weiterhin vorteilhaft gefunden, dass die paramagnetische Metallverbindung und/oder eines paramagnetischen Metalles eine Permeabilitätszahl von wenigstens 1,00002 aufweist.

Durch die erfindungsgemäßen medizinischen Arbeitsmittel ist es zum einen möglich, eine vollflächige Sichtbarkeit in der Kernspintomographie zu erreichen, wobei es jedoch auch im Rahmen der Erfindung liegt, die paramagnetische Metallverbindung und/oder eines paramagnetischen Metalles nur in Teilabschnitte des medizinischen Arbeitsmittels einzubringen oder als komplette Umhüllung für die medizinischen Arbeitsmittel.

Durch die Verwendung der erfindungsgemäßen medizinischen Arbeitsmittel besteht erstmals die Möglichkeit, die Baugrößen und Abmessungen so zu minimieren, dass trotz reduzierter Herstellungskosten ein präzises Arbeiten durch das medizinische Personal erreichbar ist, ohne Einbußen bei der Patientensicherheit hinzunehmen.

Durch erfindungsgemäßen medizinischen Arbeitsmittel ist die Produktgestaltung für die Hersteller und auch die Anwender wesentlich flexibler, wobei es noch wesentlich breitere Anwendungsmöglichkeiten in der Therapie und Diagnostik gibt, die durch den Einsatz der erfindungsgemäßen Medizin erst erschlossen werden müssen.

Die vorteilhaften Eigenschaften der erfindungsgemäßen medizinischen Arbeitsmittel sind durch die Zugabe von Gadolinium-III-Oxid erreichbar, wobei auch Mangan-II-Oxid einsetzbar ist.

Eine weitere paramagnetische Metallverbindung, welche die erfindungsgemäßen Eigenschaften der medizinischen Arbeitsmittel erzeugt, ist Eisen-II-Oxid bzw. Eisen-III-Oxid.

Bei diesen Materialien hatte sich als vorteilhaft erwiesen, dass bereits die Zugabe geringer Gewichtsprozente zu einer optimalen Erkennung der medizinischen Arbeitsmittel in der Kernspintomographie führen.

Ebenso vorteilhaft hat sich der Einsatz einer paramagnetischen Metallverbindung in Form von Legierungen herausgestellt, welche ebenfalls zur optimalen und artefaktfreien Sichtbarkeit der medizinischen Arbeitsmittel in der Kernspintomographie beitragen, wobei diese Legierungen auf Basis von Platin, Aluminium bzw. Titan herstellbar sind.

Weiterhin überraschend festgestellt wurde die Tatsache, dass durch den Einsatz bestimmter paramagnetischer Metallverbindungen und/oder paramagnetischer Metalle die medizinischen Arbeitsmittel sowohl in der Kernspintomographie als auch im Röntgen sichtbar sind. Hier kann durch Zugabe von Gadolinium III-Oxid, aber auch von Platin mit Permeabilitätszahlen von wenigstens 1.00002 die artefaktfreie Abbildung in beiden Verfahren realisiert werden.
Diese reduziert neben den Herstellungskosten für die erfindungsgemäßen medizinischen Arbeitsmittel auch deren Lagerhaltungskosten. Ein weiterer vorteilhafter Aspekt liegt darin begründet, dass keine Verwechslungsgefahr und damit eine potentielle Patientengefährdung besteht.

In einer weiteren vorteilhaften Ausführungsform der erfindungsgemäßen medizinischen Arbeitsmittel ist dem polymeren Material ein paramagnetisches Metall zugesetzt wie Platin, welches sowohl zu einer artefaktfreien und optimalen Abbildung in der Kernspintomographie als auch zusätzlich im Röntgen führt.
Es liegt jedoch im Rahmen der Erfindung, auch beliebige Kombinationen von paramagnetischen Metallverbindungen und paramagnetischen Metallen einzumischen.

Ein weiterer Vorteil der Erfindung wird darin gesehen, dass durch die Verwendung von polymeren Materialien für die erfindungsgemäßen medizinischen Arbeitsmittel sehr einfach aufgebaute und auch flexible medizinische Arbeitsmittel, insbesondere Katheter und Schläuche, welche beispielsweise durch die Verfahren der Extrusion bzw. Koextrusion herstellbar sind.

Im Folgenden werden die erfindungsgemäßen medizinischen Arbeitsmittel an diesen nicht einschränkenden Beispielen beschrieben. Diese setzen sich, bezogen auf 100 Gew.-%, wie folgt zusammen:

| | |
|---|---|
| 1. Beispiel | Thermoplastisches Polyurethan |
| | 30 Gew.- % Gadolinium-III-Oxid (Gd₂O₃) |
| 2. Beispiel | Silikonkautschuk mittels 1 % 2,4 Dichlorbenzoylperoxid vernetzt |
| | 20 Gew.-% Mangan-II-Oxid (MnO) |
| | 5 Gew.-% Gadolinium-III-Oxid (Gd₂O₃) |
| 3. Beispiel | Polypropylen |
| | 8,5 Gew.-% Eisen-II-Oxid (FeO) |
| | 2,0 Gew.-% Titan |
| 4. Beispiel | Polyvinylchlorid (weich) |
| | 25 Gew.-% Weichmacher DEHP |
| | 8 Gew.-% Epoxidiertes Sojabohnenöl |
| | 2 Gew.-% Stabilisatoren |
| | 10 Gew.-% Eisen-III-Oxid (Fe₂O₃) |
| 5. Beispiel | Polyethylen |
| | 5 Gew.-% Titanlegierung mit Aluminium und Vanadium |

## Patentansprüche

1. Zusammensetzung geeignet für die Herstellung von medizinischen Arbeitsmittel, insbesondere Katheter, Schläuche, Rundschnüre, Formteile, umfassend
(A) 100 Gewichtsteile eines Polymers
(B) 0,1 bis 80 Gewichtsteile wenigstens einer paramagnetischen Metallverbindung und oder
(C) 0,1 bis 80 Gewichteile wenigstens eines paramagnetischen Metalles

2. Zusammensetzung nach Anspruch 1, **dadurch gekennzeichnet, dass** die paramagnetische Metallverbindung und/oder das paramagnetische Metall eine Permeabilitätszahl von wenigstens 1,00002 aufweist.

3. Zusammensetzung nach Anspruch 1 bis 2, wobei Komponente (B) Gaolinium-III-Oxid (Gd₂O₃) ist.

4. Zusammensetzung nach Anspruch 1 bis 2, wobei Komponente (B) Mangan-II-Oxid (MnO) ist.

5. Zusammensetzung nach Anspruch 1 bis 2, wobei Komponente (B) Eisen-II-Oxid (FeO) ist.

6. Zusammensetzung nach Anspruch 1 bis 2, wobei Komponente (B) Eisen-III-Oxid (Fe₂O₃) ist.

7. Zusammensetzung nach Anspruch 1 bis 2, wobei Komponente (B) eine Platinlegierung ist.

8. Zusammensetzung nach Anspruch 1 bis 2, wobei Komponente (B) eine Titanlegierung ist.

9. Zusammensetzung nach Anspruch 1 bis 2, wobei Komponente (B) eine Aluminiumlegierung ist.

10. Zusammensetzung nach Anspruch 1 bis 2 , wobei die Komponente (C) Platin ist.

11. Zusammensetzung nach Anspruch 1 bis 2 , wobei die Komponente (C) Titan ist.

12. Zusammensetzung nach Anspruch 1 bis 2 , wobei die Komponente (C) Aluminium ist.

13. Medizinische Arbeitsmittel, insbesondere Katheter, Schläuche, Rundschnüre, Formteile aus polymeren Materialien, wobei diese Arbeitsmittel eine besondere Ausstattung zu ihrer Erkennung in der Kernspintomographie aufweisen,
**dadurch gekennzeichnet,**
**dass** in das polymere Material vor der eigentlichen Formgebung eine definierte Menge wenigstens einer paramagnetischen Metallverbindung und/oder eines paramagnetischen Metalles eingemischt ist.

14. Medizinische Arbeitsmittel nach Anspruch 13, **dadurch gekennzeichnet, dass** die Menge der paramagnetischen Metallverbindung und/oder eines paramagnetischen Metalles zwischen 0,1 und 80 Gewichtsprozent liegt, vorzugsweise zwischen 1 und 40 Gewichtsprozent.

15. Medizinische Arbeitsmittel nach den Ansprüchen 13 bis 14, **dadurch gekennzeichnet, dass** die paramagnetische Metallverbindung und/oder eines paramagnetischen Metalles eine Permeabilitätszahl von wenigstens 1,00002 aufweist.

16. Medizinische Arbeitsmittel nach einem der Ansprüche 13 bis 15, **dadurch gekennzeichnet, dass** die paramagnetische Metallverbindung Gadolinium-III-Oxid (Gd₂O₃) ist.

17. Medizinische Arbeitsmittel nach einem der Ansprüche 13 bis 16, **dadurch gekennzeichnet, dass** die paramagnetische Metallverbindung Mangan-II-Oxid (MnO) ist.

18. Medizinische Arbeitsmittel nach einem der Ansprüche 13 bis 17, **dadurch gekennzeichnet, dass** die paramagnetische Metallverbindung Eisen-II-Oxid (FeO) ist.

19. Medizinische Arbeitsmittel nach einem der Ansprüche 13 bis 18, **dadurch gekennzeichnet, dass** die paramagnetische Metallverbindung Eisen-III-Oxid (Fe₂O₃) ist.

20. Medizinische Arbeitsmittel nach einem der Ansprüche 13 bis 19, **dadurch gekennzeichnet, dass** die paramagnetische Metallverbindung eine Platinlegierung ist.

21. Medizinische Arbeitsmittel nach einem der Ansprüche 13 bis 20, **dadurch gekennzeichnet, dass** die paramagnetische Metallverbindung eine Titanlegierung ist.

22. Medizinische Arbeitsmittel nach einem der Ansprüche 13 bis 21, **dadurch gekennzeichnet, dass** die paramagnetische Metallverbindung eine Aluminiumlegierung ist.

23. Medizinische Arbeitsmittel, insbesondere Katheter, Schläuche, Rundschnüre, Formteile aus polymeren Materialien, wobei diese Arbeitsmittel eine besondere Ausstattung zu ihrer Erkennung in der Kernspintomographie und im Röntgen aufweisen, **dadurch gekennzeichnet, dass** in das Ausgangspolymere vor der eigentlichen Formgebung eine definierte Menge wenigstens einer paramagnetischen Metallverbindung und/oder eines paramagnetischen Metalles eingemischt ist.

24. Medizinische Arbeitsmittel nach Anspruch 23, **dadurch gekennzeichnet, dass** die Menge der paramagnetischen Metallverbindung und/oder des paramagnetischen Metalles zwischen 0,1 und 80 Gewichtsprozent liegt, vorzugsweise zwischen 1 und 40 Gewichtsprozent.

25. Medizinische Arbeitsmittel nach einem der Ansprüche 23 bis 24, **dadurch gekennzeichnet, dass** die paramagnetische Metallverbindung ein Gadolinium-III-Oxid (Gd₂O₃) ist.

26. Medizinische Arbeitsmittel nach einem der Ansprüche 23 bis 25, **dadurch gekennzeichnet, dass** die paramagnetische Metallverbindung ein Platin ist.

27. Verwendung eines polymeren Materials mit einem Zusatz von 0,1 bis 80 Gewichtsprozent wenigstens einer paramagnetischen Metallverbindung, wobei dieser eine Permeabilitätszahl von wenigstens 1,00002 aufweist, zur Herstellung von medizinischen Arbeitsmitteln wie Katheter, Schläuche, Rundschnüre, Formteile und dergleichen.
